Europäisches Patentamt

European Patent Office

Office européen des brevets

(11) Veröffentlichungsnummer: **0 257 199 B1**

(12) **EUROPÄISCHE PATENTSCHRIFT**

(45) Veröffentlichungstag der Patentschrift: **11.11.92**

(51) Int. Cl.5: **A61B 17/22**, A61B 6/02

(21) Anmeldenummer: **87107207.0**

(22) Anmeldetag: **18.05.87**

(54) **Einrichtung zum Zertrümmern von Konkrementen.**

(30) Priorität: **18.07.86 DE 3624374**

(43) Veröffentlichungstag der Anmeldung:
**02.03.88 Patentblatt 88/09**

(45) Bekanntmachung des Hinweises auf die
Patenterteilung:
**11.11.92 Patentblatt 92/46**

(84) Benannte Vertragsstaaten:
**DE FR GB NL**

(56) Entgegenhaltungen:
**EP-A- 0 139 941      EP-A- 0 168 559**
**EP-A- 0 225 104      DE-A- 2 655 661**
**DE-A- 3 146 628      GB-A- 2 002 987**
**GB-A- 2 098 425**

**NEUE ZÜRCHER ZEITUNG, Nr. 119, 28. Mai**
**1986, Seite 51; H. CERUTTI:**
**"Nierensteinzertrümmerer der zweiten Gene-**
**ration"**

(73) Patentinhaber: **SIEMENS AKTIENGESELL-**
**SCHAFT**
**Wittelsbacherplatz 2**
**W-8000 München 2(DE)**

(72) Erfinder: **Grasser, Franz, Dipl.-Ing.**
**Neuwiesenstrasse 27**
**W-8557 Eggolsheim(DE)**
Erfinder: **Rattner, Manfred**
**Am Eichengarten 8**
**W-8520 Erlangen(DE)**

## Beschreibung

Die Erfindung betrifft eine Einrichtung zum Zertrümmern von im Körper eines Lebewesens befindlichen Konkrementen mit einem Stoßwellengenerator und mit einer Untersuchungsvorrichtung zur Ortung der Konkremente, die wenigstens einen Bildspeicher aufweist, in dem ein Bild eingespeichert wird.

In der DE-OS 31 22 056 ist eine derartige Vorrichtung beschrieben, die beispielsweise zum Zertrümmern von Harn-, Nieren-, Gallensteinen oder dergleichen dient. In einer Fokussierungskammer wird als Stoßwellengenerator eine Stoßwelle z. B. durch Funkenentladung erzeugt, die auf das Konkrement konzentriert wird und es zerkleinert. Damit genau festgestellt werden kann, ob sich das Konkrement im Brennpunkt der Fokussierungskammer befindet, ist die Einrichtung zur Ortung mit einer Röntgenuntersuchungsvorrichtung verbunden. Im Durchleuchtungsbetrieb werden die einzelnen Bilder eines Stereobildpaares in Bildspeicher einzeln oder über mehrere Bilder integriert im Fernsehtakt eingespeichert. Hierbei tritt aber das Problem auf, daß insbesondere bei der Behandlung von Nierensteinen diese während der Atmung bewegt werden, so daß eine genaue Lokalisierung der Konkremente zum Zeitpunkt der Auslösung nicht erfolgen kann. Das gleiche Problem tritt ebenfalls bei der Verwendung von Ultraschall-Untersuchungsvorrichtungen auf.

Eine Einrichtung der eingangs genannten Art ist außerdem in der EP-A-0 168 559 beschrieben. Im Falle dieser Einrichtung sind zwei Untersuchungsvorrichtungen zur Ortung der Konkremente vorhanden, die miteinander korreliert sind und die Informationen für eine Echtzeit-Positionskorrektur liefern, mit dem Ziel, sicherzustellen, daß sich das Konkrement zum Zeitpunkt der Stoßwellenauslösung im Fokus der Stoßwellen befindet.

Die Erfindung geht von der Aufgabe aus, eine Vorrichtung der eingangs genannten Art zu schaffen, die es ermöglicht, eine sehr genaue Lokalisierung der Konkremente in bezug auf den Zeitpunkt der Auslösung der Stoßwellen durchzuführen, so daß eine sichere, gewebeschonende Zerkleinerung der Konkremente erfolgt.

Die Aufgabe wird erfindungsgemäß dadurch gelöst, daß eine Vergleichsvorrichtung für das gespeicherte und ein dem gespeicherten zeitlich nachfolgendes Bild vorgesehen ist, die mit einer Einrichtung zur Auslösung der Stoßwellen verbunden ist, und daß Stoßwellen bei weitgehender Gleichheit in bezug auf den Vergleich des gespeicherten und des zeitlich nachfolgenden Bildes auslösbar sind. Dadurch wird erreicht, daß beispielsweise bei Gleichheit des gespeicherten und des aktuellen Röntgen- oder Ultraschallbildes Stoßwellen ausgelöst werden können. Eine Gleichheit ist aber nur dann gegeben, wenn das Untersuchungsobjekt keine Bewegung, hervorgerufen beispielsweise durch Atmung oder durch den Herzschlag, gegenüber dem Zeitpunkt der Einspeicherung des ersten Bildes durchgeführt hat. Es wird somit eine sehr genaue Übereinstimmung des Lokalisationspunktes mit dem Brennpunkt des Stoßwellengenerators erreicht, so daß die gesamte aufgewandte Energie zur Zerkleinerung des Konkrements genutzt werden kann.

Eine Bewegung insbesondere des zu behandelnden Konkrementes wird erkannt, wenn Auswahlmittel vorhanden sind, die mit der Untersuchungsvorrichtung derart verbunden sind, daß ein Vergleich nur in einem besonders interessierenden Gebiet durchgeführt wird. Geringfügige Bewegungen bleiben unberücksichtigt, wenn an der Vergleichsvorrichtung eine Auswerteschaltung angeschlossen ist, die ein Signal erzeugt, das der Anzahl der gleichen oder der ungleichen Bildpunkte entspricht, und wenn die Auswerteschaltung mit einer Schwellenwertschaltung verbunden ist, die eine Auslösung von Stoßwellen nur ermöglicht, wenn sich das Signal oberhalb bzw. unterhalb einer bestimmten einstellbaren Schwelle befindet. Die Auslösung mehrerer Stoßwellen in einer Bewegungsphase wird ermöglicht, wenn ein Detektor für eine Phase der minimalen Bewegung der Konkremente vorhanden ist und wenn das Ausgangssignal des Detektors den Speichervorgang des Bildspeichers steuert.

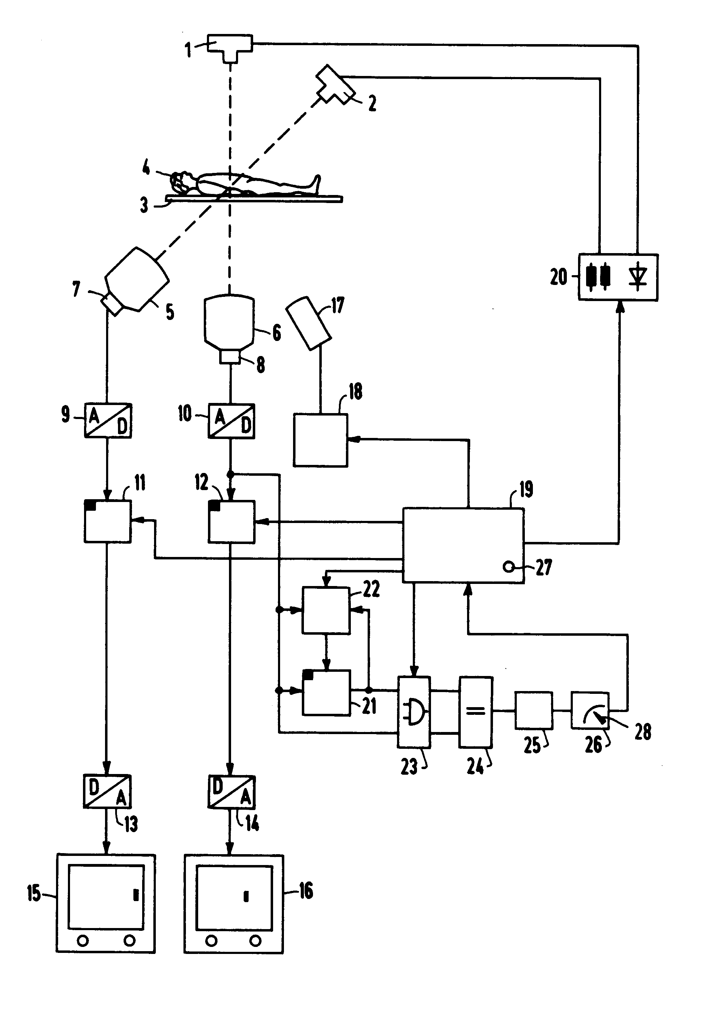

Die Erfindung ist nachfolgend anhand eines in der Zeichnung dargestellten Ausführungsbeispieles näher erläutert.

In der Figur ist eine erfindungsgemäße Einrichtung zum Zertrümmern von im Körper eines Lebewesens befindlichen Konkrementen mit einer Röntgenuntersuchungsvorrichtung dargestellt, die zwei Röntgenröhren 1 und 2 aufweist, die Röntgenstrahlenbündel erzeugen, die eine auf einer Patientenliege 3 befindliche, zu behandelnde Untersuchungsperson 4 durchdringen und auf die Eingangsleuchtschirme von Röntgenbildverstärkern 5 und 6 fallen. Die Röntgenröhre 1 und der Röntgenbildverstärker 6 sind beispielsweise derart angeordnet, daß der Zentralstrahl des Röntgenstrahlenbündels der Röntgenröhre 1 senkrecht auf die Untersuchungsperson 4 fällt (a.p.-Projektion). Die Röntgenröhre 2 und der Röntgenbildverstärker 5 können derart schräg angeordnet sein, daß der Zentralstrahl der Röntgenröhre 2 den Zentralstrahl der Röntgenröhre 1 in einem Zielgebiet innerhalb der Untersuchungsperson 4 unter einem Winkel von beispielsweise 45° schneidet (c.c.-Projektion). Dadurch erhält man Durchleuchtungsbilder aus zwei verschiedenen Projektionsrichtungen, so daß man die Untersuchungsperson 4 durch die Patientenliege 3 derart verschieben kann, daß sich die Konkremente in dem Zielgebiet einer Vorrichtung zum Zertrümmern von Konkrementen befinden.

Die Ausgangssignale der an den Röntgenbildverstärkern 5 und 6 angekoppelten Fernsehkameras 7 und 8 werden über zwei Analog/Digital-Wandler (A/D-Wandler 9, 10 ) in zwei Bildspeicher 11 und 12 eingelesen. Die Ausgangssignale der beiden Bildspeicher 11 und 12 können über zwei Digital/Analog-Wandler (D/A-Wandler 13, 14) auf zwei Monitoren 15, 16 betrachtet werden. Befinden sich nun die Konkremente im Zielgebiet, so erscheinen sie in der Mitte der Bildschirme der Monitore 15 und 16.

Zur Zerkleinerung der in dem Zielgebiet befindlichen Konkremente ist ein Stoßwellengenerator 17 vorgesehen, der in der Figur schematisch dargestellt ist. Er erzeugt in bekannter Weise Stoßwellen, die zu einer Zertrümmerung der Konkremente führen, wenn sie beispielsweise im Brennpunkt des Stoßwellengenerators 17 liegen. Die Untersuchungseinrichtung und der Stoßwellengenerator 17 sind derart fest miteinander verbunden, daß die Stoßwellen im Zielgebiet der Röntgenuntersuchungseinrichtung fokussiert sind. Der Stoßwellengenerator 17 ist an einer Ansteuerschaltung 18 angeschlossen. Eine Steuervorrichtung 19 ist mit einem Hochspannungsgenerator 20 zur Steuerung der Röntgenröhren 1 und 2, mit dem Bildspeichern 11 und 12 und der Ansteuerschaltung 18 für den Stoßwellengenerator 17 verbunden.

Das Ausgangssignal des A/D-Wandlers 10 wird einem weiteren Bildspeicher 21 zugeführt, dessen Steuereingang über einen Detektor 22 für die minimale Bewegung der Konkremente mit der Steuervorrichtung 19 verbunden ist. Dem Detektor 22 wird weiterhin das aktuelle Videosignal vom A/D-Wandler 10 und das gespeicherte vom Bildspeicher 21 zugeführt. Die Ausgänge des Bildspeichers 21 und des A/D-Wandlers 10 sind über eine Auswahlschaltung 23 mit einer Vergleichsvorrichtung 24 verbunden. Das Ausgangssignal der Vergleichsvorrichtung 24 steuert über eine Auswerteschaltung 25 und eine Schwellenwertschaltung 26 die Steuervorrichtung 19.

Zu Beginn der Untersuchung werden die Röntgenröhren 1 und 2 eingeschaltet, so daß von der Untersuchungsperson 4 Röntgenbilder erstellt und in den Bildspeichern 11 und 12 abgespeichert werden. Die Röntgenbilder werden auf den Monitoren 15 und 16 beobachtet. Nunmehr wird durch Verschieben der Patientenliege 3 die Untersuchungsperson 4 derart ausgerichtet, daß sich die zu zerkleinernden Konkremente in dem Zielgebiet befinden. Dies kann dadurch festgestellt werden, daß sich die Konkremente in der Mitte der Bildschirme der Monitore 15 und 16 befinden. Diese Ausrichtung kann im Durchleuchtungsbetrieb erfolgen.

Nach erfolgter Ausrichtung kann beispielsweise durch Betätigung des Knopfes 27 die Einspeicherung eines Röntgenbildes in den Bildspeicher 21 dadurch eingeleitet werden, daß die Steuervorrichtung 19 den Detektor 22 freigibt. Der Detektor 22 ermöglicht nunmehr die Einspeicherung von aufeinanderfolgenden Röntgenbildern in den Bildspeicher 21. Durch Vergleich des gespeicherten und des aktuellen Videosignales werden durch den Detektor 22 die Phase mit der geringsten Bewegung der Konkremente ermittelt und der Einspeichervorgang abgeschlossen, so daß in dem Bildspeicher 21 ein Röntgenbild gespeichert ist, das der Phase der geringsten Bewegung entspricht.

Anschließend wird nach Rückmeldung durch den Detektor 22 von der Steuervorrichtung 19 die Auswahlschaltung 23 angesteuert, die die ausgewählten Bildpunkte freigibt, die einem besonders interessierenden Gebiet (roi) entsprechen. Die Auswahl kann beispielsweise in bekannter Weise durch einen nicht dargestellten Lichtgriffel auf dem Monitor 16 erfolgen. Es kann aber auch, da sich die Konkremente im Mittelpunkt des auf dem Monitor 16 dargestellten Röntgenbildes befinden, nur ein fest vorgegebener mittlerer Bereich freigegeben werden. Anstelle eines ausgewählten Bereiches können aber auch die Videosignale der vollständigen Bilder der Vergleichsvorrichtung 24 zugeführt werden.

Die Vergleichsvorrichtung 24 kann einen Komparator aufweisen, der beide Videosignale, das gespeicherte und das aktuelle, bildpunktweise vergleicht und ein entsprechendes Ausgangssignal erzeugt, das der Anzahl von gleichen und/oder ungleichen Bildpunkten entspricht. Die Vergleichsschaltung 24 kann aber auch aus einer Subtraktionsstufe bestehen, die ein Differenzsignal bei voneinander abweichenden Bildpunkten erzeugt.

Die an der Vergleichsvorrichtung 24 angeschlossene Auswerteschaltung 25 kann beispielsweise einen oder mehrere Zähler aufweisen, der die Anzahl der gleichen und/oder ungleichen Bildpunkte addiert. Überschreitet der Zählerstand, der den gleichen Bildpunkten entspricht, einen durch einen Einsteller 28 der Schwellenwertschaltung 26 eingestellten Schwellenwert, so erzeugt die Schwellenwertschaltung ein Freigabesignal für den Stoßwellengenerator 17. Es kann aber auch die Anzahl der ungleichen Bildpunkte ausgewertet werden, wenn die Schwellenwertschaltung 26 ein Freigabesignal erzeugt, das unterhalb der durch den Einsteller 28 eingestellten Schwelle liegt. Das von der Schwellenwertschaltung 26 erzeugte Freigabesignal wird der Steuervorrichtung 19 zugeführt, die die Ansteuerschaltung 18 des Stoßwellengenerators 17 ansteuert.

Durch diese Vorrichtung wird erreicht, daß Stoßwellen nur dann ausgelöst werden, wenn die Konkremente sich in ihrer geringsten Bewegungsphase befinden.

Soll die Röntgenuntersuchungseinrichtung nur im reinen Durchleuchtungsbetrieb betrieben werden, so können die Bildspeicher 11 und 12 entfallen. Es kann aber auch nur der Bildspeicher 12 entfallen, wenn das Ausgangssignal des Bildspeichers 21 dem D/A-Wandler 14 und somit dem Monitor 16 zugeführt wird. Eine Kontrolle der Bewegung in verschiedenen Richtungen kann erfolgen, wenn an dem A/D-Wandler 9 der zweiten Projektion der gleiche Schaltungsaufbau 21 bis 28 wie an dem A/D-Wandler 10 angeschlossen ist.

Anstelle der Auskopplung aus dem Videosignal der Röntgenuntersuchungseinrichtung zur Gewinnung der Triggerimpulse für die Auslösung der Stoßwellen können die Triggerimpulse auch aus dem Bildsignal einer Ultraschall-Untersuchungseinrichtung gewonnen werden. Dadurch wird die dem Patienten applizierte Röntgendosis erspart. Es kann aber auch auf eine getrennte Ultraschall-Untersuchungseinrichtung verzichtet werden, wenn zur Erzeugung des für den Vergleich herangezogenen Bildes die aus der von der Stoßwelle am Stein reflektierte mechanische Energie verwendet wird. Dadurch lassen sich die Kosten für die Gesamtanlage vermindern.

## Patentansprüche

1. Einrichtung zum Zertrümmern von im Körper eines Lebewesens befindlichen Konkrementen mit einem Stoßwellengenerator und mit einer Untersuchungsvorrichtung (1 bis 16, 19 bis 28) zur Ortung der Konkremente, die wenigstens einen Bildspeicher (21) aufweist, in dem ein Bild eingespeichert wird, **dadurch gekennzeichnet,** daß eine Vergleichsvorrichtung (24) für das gespeicherte und ein dem gespeicherten zeitlich nachfolgendes Bild vorgesehen ist, die mit einer Einrichtung zur Auslösung der Stoßwellen verbunden ist, und daß Stoßwellen bei weitgehender Gleichheit in bezug auf den Vergleich des gespeicherten und des zeitlich nachfolgenden Bildes auslösbar sind.

2. Einrichtung nach Anspruch 1, **dadurch gekennzeichnet,** daß Auswahlmittel (23) vorhanden sind, die mit der Untersuchungsvorrichtung (1 bis 16, 19 bis 28) derart verbunden sind, daß ein Vergleich nur in einem besonders interessierenden Gebiet durchgeführt wird.

3. Einrichtung nach Anspruch 1 oder 2, **dadurch gekennzeichnet,** daß an der Vergleichsvorrichtung (24) eine Auswerteschaltung (25) angeschlossen ist, die ein Signal erzeugt, das der Anzahl der ungleichen Bildpunkte entspricht, und daß die Auswerteschaltung (25) mit einer Schwellenwertschaltung (26) verbunden ist, die eine Auslösung von Stoßwellen nur ermöglicht, wenn sich das Signal unterhalb einer bestimmten einstellbaren Schwelle befindet.

4. Einrichtung nach Anspruch 1 oder 2, **dadurch gekennzeichnet,** daß an der Vergleichsvorrichtung (24) eine Auswerteschaltung (25) angeschlossen ist, die ein Signal erzeugt, das der Anzahl der gleichen Bildpunkte entspricht, und daß die Auswerteschaltung (25) mit einer Schwellenwertschaltung (26) verbunden ist, die eine Auslösung von Stoßwellen nur ermöglicht, wenn sich das Signal oberhalb einer bestimmten einstellbaren Schwelle befindet.

5. Einrichtung nach einem der Ansprüche 1 bis 4, **dadurch gekennzeichnet,** daß ein Detektor (22) für eine Phase der minimalen Bewegung der Konkremente vorhanden ist und daß das Ausgangssignal des Detektors (22) den Speichervorgang des Bildspeichers (21) steuert.

## Claims

1. Device for disintegrating calculi in the body of a living being, having a shock wave generator and an examination device (1 to 16, 19 to 28) for locating the calculi which has at least one image memory (21) in which an image is stored, characterised in that there is provided a comparator (24) for the stored image and an image temporally following the stored image, which is connected to a device for releasing the shock waves, and in that shock waves can be triggered upon substantial uniformity with respect to the comparison of the stored image and subsequent image.

2. Device according to claim 1, characterised in that selection means (23) are present which are connected to the examination device (1 to 16, 19 to 28) in such a way that a comparison is carried out only in a region of particular interest.

3. Device according to claim 1 or 2, characterised in that an evaluation circuit (25) is connected to the comparator (24) and produces a signal which corresponds to the number of non-identical image points, and in that the evaluation circuit (25) is connected to a threshold value circuit (26) which makes possible triggering of shock waves only when the signal is below a specific adjustable threshold.

4. Device according to claim 1 or 2, characterised in that an evaluation circuit (25) is connected to the comparator (24) and produces a signal which corresponds to the number of identical image points, and in that the evaluation circuit (25) is connected to a threshold value circuit (26) which makes possible triggering of shock waves only when the signal is above a specific adjustable threshold.

5. Device according to one of claims 1 to 4, characterised in that there is a detector (22) for a phase of minimal movement of the calculi, and in that the output signal of the detector (22) controls the storage process of the image memory (21).

**Revendications**

1. Dispositif pour détruire des concrétions situées dans le corps d'un être vivant, comportant un générateur d'ondes de choc et un dispositif d'examen (1 à 16, 19 à 28) pour localiser les concrétions, et qui possède au moins une mémoire d'images (21) dans laquelle est mémorisée une image, caractérisé par le fait qu'il est prévu un dispositif comparateur (24) pour comparer une image mémorisée et une image suivant l'image mémorisée et qui est raccordé à un dispositif servant à déclencher les ondes de choc, et des ondes de choc peuvent être déclenchées dans le cas où il existe une large ressemblance sur la base de la comparaison entre l'image mémorisée et l'image suivante.

2. Dispositif suivant la revendication 1, caractérisé par le fait qu'il est prévu des moyens de sélection (23) qui sont raccordés au dispositif d'examen (1 à 16, 19 à 28), en sorte qu'une comparaison est exécutée uniquement dans un domaine particulièrement intéressant.

3. Dispositif suivant la revendication 1 ou 2, caractérisé par le fait qu'au dispositif comparateur (24) est raccordé un circuit d'évaluation (25) qui produit un signal qui correspond au nombre des points images non identiques et que le circuit d'évaluation (25) est raccordé à un circuit à valeur de seuil (26), qui permet un déclenchement d'ondes de choc uniquement lorsque le signal est inférieur à un seuil déterminé réglable.

4. Dispositif suivant la revendication 1 ou 2, caractérisé par le fait qu'au dispositif comparateur (24) est raccordé un circuit d'évaluation (25) qui produit un signal correspondant au nombre des points images identiques, et que le circuit d'évaluation (25) est raccordé à un circuit à valeur de seuil (26), qui permet un déclenchement d'ondes de choc uniquement lorsque le signal se situe au-dessus d'un seuil réglable déterminé.

5. Dispositif suivant l'une des revendications 1 à 4, caractérisé par le fait qu'un détecteur (22) est présent pour une phase du déplacement minimum des concrétions et que le signal de sortie du détecteur (22) commande l'opération de mémorisation dans la mémoire d'images (21).